(19) Europäisches Patentamt
European Patent Office
Office européen des brevets

(11) Numéro de publication : **0 387 116 B1**

# (12) FASCICULE DE BREVET EUROPEEN

(45) Date de publication du fascicule du brevet : **08.12.93 Bulletin 93/49**

(51) Int. Cl.$^5$ : **C07C 57/66,** C07C 51/60

(21) Numéro de dépôt : **90400453.8**

(22) Date de dépôt : **19.02.90**

## (54) Procédé de fabrication de chlorure d'acryloyle.

(30) Priorité : **06.03.89 FR 8902918**

(43) Date de publication de la demande : **12.09.90 Bulletin 90/37**

(45) Mention de la délivrance du brevet : **08.12.93 Bulletin 93/49**

(84) Etats contractants désignés : **AT BE CH DE DK ES FR GB GR IT LI LU NL SE**

(56) Documents cités :
**EP-A- 0 166 293**
**US-A- 1 921 767**
**US-A- 1 963 749**

(73) Titulaire : **ELF ATOCHEM S.A.**
**4 & 8, Cours Michelet La Défense 10**
**F-92800 Puteaux (FR)**

(72) Inventeur : **Grosius, Paul**
**11, rue Jeanne d'Arc**
**F-57540 Petite Rosselle (FR)**
Inventeur : **Croizy, Jean-François**
**84, rue Principale**
**F-5740 Farschviller (FR)**

(74) Mandataire : **Rochet, Michel**
**ELF ATOCHEM S.A. Département Propriété Industrielle 4-8, Cours Michelet La Défense 10 - Cedex 42**
**F-92091 Paris-La-Défense (FR)**

**EP 0 387 116 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention porte sur un procédé de fabrication de chlorure d'acryloyle par réaction de l'acide acrylique sur le phénylchloroforme.

Le chlorure d'acryloyle constitue un intermédiaire de synthèse très réactif. Les voies d'accès à ce chlorure d'acide, par l'intermédiaire des réactifs classiques, comme le phosgène, le chlorure de sulfonyle, les tri- ou pentachlorures de phosphore, etc., représentent une technologie complexe, compte tenu de la formation d'HCl et de la réactivité de la double liaison acrylique. Il en résulte une chute de rendement non négligeable. On connaît cependant, une autre voie d'accès au chlorure d'acryloyle, basée sur la réaction de l'acide acrylique avec le phénylchloroforme, en présence d'un acide de Lewis comme catalyseur et d'un inhibiteur de polymérisation de l'acide acrylique, le chlorure de benzoyle étant également formé par la réaction. Le schéma réactionnel de cette dernière, qui consiste en une solvolyse du phénylchloroforme par l'acide acrylique, est le suivant :

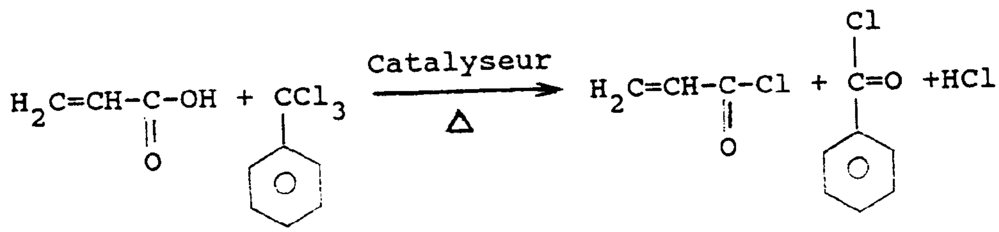


Le brevet américain US-A-3 691 217 décrit, en son exemple 19, la fabrication du chlorure d'acryloyle par cette méthode, la réaction étant conduite à une température de 100 °C et le catalyseur utilisé étant le chlorure stannique.

Divers sous-produits sont susceptibles de se former dans la réaction de l'acide acrylique sur le phénylchloroforme, par réaction entre l'acide acrylique et un chlorure d'acide (formation d'anhydride et d'HCl, favorisée par une élévation de la température), par réaction d'échange entre le chlorure de benzoyle et l'acide acrylique (formation d'acide benzoïque) et, surtout, comme indiqué ci-dessous, addition de l'acide chlorhydrique sur la double liaison acrylique. Les données cinétiques montrent en effet que l'acide acrylique réagit mieux avec HCl que son chlorure associé, et que, quel que soit le chemin réactionnel suivi, le chlorure de chloro-3 acryloyle est obtenu comme sous-produit majoritaire :

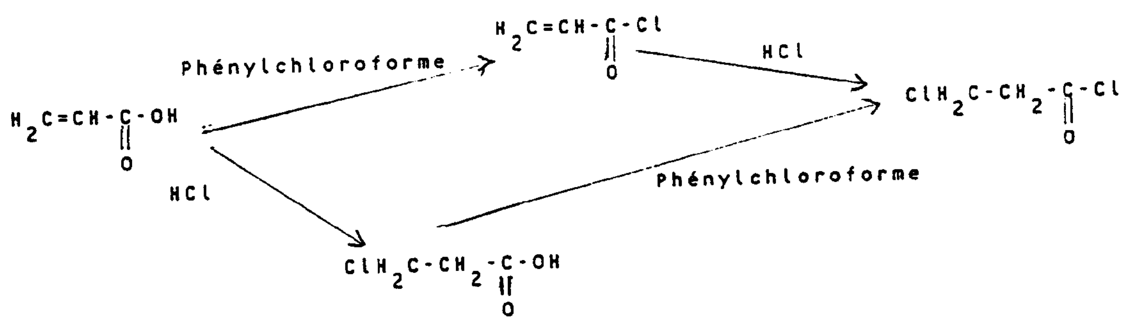


Le brevet américain précité montre bien cette production parallèle de chlorure de chloro-3 acryloyle, puisque dans son exemple 19, il est indiqué qu'il se forme 20% en poids de chlorure de chloro-3 acryloyle, quantité importante empêchant l'exploitation industrielle de ce procédé.

Pour résoudre cette difficulté et obtenir un chlorure d'acryloyle de grande pureté, avec un bon rendement, la Société déposante a découvert que cette même réaction pouvait être conduite à des températures relativement élevées, contrairement aux indications de la technique antérieure qui préconise des températures modérées quand le milieu réactionnel comprend de l'acide acrylique compte tenu de la faible stabilité thermique de ces acides, avec, par ailleurs, utilisation de catalyseurs différents de $SnCl_4$.

La présente invention a donc pour objet un procédé de fabrication de chlorure d'acryloyle par réaction de l'acide acrylique sur le phénylchloroforme, en présence d'au moins un acide de Lewis comme catalyseur et d'au moins un agent inhibiteur de polymérisation, caractérisé par le fait que le catalyseur est choisi parmi l'oxyde de zinc et le tétrachlorure de zirconium et qu'on conduit la réaction à une température d'au moins 105 °C.

On conduit généralement la réaction avec un rapport sensiblement molaire de l'acide acrylique au phénylchloroforme.

Dans un mode de réalisation préféré, on additionne l'acide acrylique au milieu constitué par le phénylchloroforme, le catalyseur et l'inhibiteur de polymérisation, et porté à une température d'au moins 105 °C, le chlorure d'acryloyle formé distillant dès le début de l'introduction de l'acide acrylique.

Compte tenu du niveau thermique élevé nécessaire selon l'invention pour obtenir un chlorure d'acryloyle de qualité élevée, et compte tenu du bas point d'ébullition de ce produit, il est indispensable d'utiliser une réfrigération très efficace d'autant plus nécessaire que ce produit est lacrymogène.

L'addition de l'acide acrylique s'effectue, de préférence, par le fond du réacteur, pour éviter une perte de réactif par entraînement avec HCl, pour favoriser la réaction de l'acide acrylique et du phénylchloroforme, et pour limiter l'addition d'HCl sur la double liaison acrylique par suite d'une concentration en HCl plus faible. Le chlorure d'acryloyle formé est récupéré en sortie d'une colonne de distillation montée sur la sortie du réacteur, à la partie supérieure de celui-ci. L'acide acrylique est introduit à une vitesse qui est fonction de l'appareillage utilisé, et la facilité à éliminer l'acide chlorhydrique formé et à condenser le chlorure d'acryloyle volatil.

De préférence, on conduit la réaction selon l'invention à une température comprise entre 105 °C et 180 °C. En particulier, on maintient la température choisie tout au long de l'addition de l'acide acrylique, puis on l'élève progressivement pour permettre l'achèvement de la distillation du chlorure d'acryloyle. Une fois cette distillation achevée, on recueille le chlorure de benzoyle restant par distillation, de préférence sous pression réduite. La réaction de l'acide acrylique sur le phénylchloroforme est, quant à elle, généralement conduite à la pression atmosphérique ; elle pourrait cependant être effectuée à une pression inférieure à la pression atmosphérique.

La durée de la réaction selon l'invention dépend de la quantité de catalyseur et de la vitesse d'introduction de l'acide acrylique. Elle se situe notamment entre 0,5 et 5 heures.

Par ailleurs, on utilise de façon habituelle une quantité de catalyseur et une quantité d'inhibiteur de polymérisation se situant respectivement entre environ 0,1 et 5% en poids, et environ 0,05 à 2 % en poids, par rapport à la quantité d'acide acrylique mise en jeu.

L'hydroquinone, l'éther méthylique de l'hydroquinone, le ditert.-butyl paracrésol, la phénothiazine et le chlorure de cuivre sont des exemples d'inhibiteurs de polymérisation que l'on peut utiliser conformément à l'invention. Par ailleurs, le fait de conduire la réaction de l'invention en présence d'air assure déjà une inhibition de la polymérisation de l'acide acrylique.

Les exemples suivants sont destinés à mieux illustrer la présente invention, sans toutefois en limiter la portée. Dans ces exemples, les pourcentages sont exprimés en poids. L'exemple 5 est un exemple comparatif.

Exemples 1 à 5 :

Mode opératoire général

Les paramètres qui varient d'un exemple à l'autre et les résultats obtenus figurent dans le Tableau ci-après.

Dans un réacteur d'une capacité de 1 litre, à double enveloppe, équipé d'un dispositif d'agitation mécanique centrale, d'une colonne adiabatique (15 plateaux théoriques environ) et de réfrigérants ascendants très efficaces, on introduit 0,14 g d'hydroquinone (0,1% en poids par rapport à l'acide acrylique à introduire), une quantité donnée d'oxyde de zinc ou de tétrachlorure de zirconium comme catalyseur et 399,2 g de phénylchloroforme (2 moles ; pureté : 97,9%).

On porte l'ensemble à la température indiquée. Lorsque cette température est atteinte, on additionne, en fond de réacteur, en l'espace de 70 minutes environ, par l'intermédiaire d'une pompe doseuse, 144 g d'acide acrylique glacial (2 moles).

La température indiquée est maintenue tout au long de l'addition de l'acide acrylique, lequel distille simultanément à pression atmosphérique (température de la tête de colonne : environ 51 °C). Puis, après l'achèvement de l'addition de l'acide acrylique, la température est progressivement élevée jusqu'à environ 180 °C pour permettre une distillation totale du chlorure d'acryloyle. L'acide chlorhydrique, qui se forme simultanément au cours de la réaction, est piégé par barbotage dans l'eau.

En fin de réaction, le chlorure de benzoyle formé est distillé sous pression réduite (température de tête de colonne : environ 100 °C ; pression : 6,7 x $10^3$Pa).

Tableau

| Exemple | Catalyseur | Température °C | Chlorure d'acryloyle (mole**) | Chlorure de chloro-3 acryloyle (mole**) |
|---|---|---|---|---|
| 1 | ZnO 0,72 g | 110 | 0,74 | 0,01 |
| 2 | ZnO 0,72 g | 162 | 0,74 | 0,005 |
| 3 | $ZrCl_4$ 0,72 g | 120 | 0,78 | 0,005 |
| 4 | $ZrCl_4$ 2,06 g | 120 | 0,78 | 0,005 |
| 5* (comp.) | ZnO 0,72 g | 92 | 0,61 | 0,36 |

* La réaction a duré 1 h et a été conduite en deux étapes : la réaction proprement dite et la distillation des produits formés.

** Par mole d'acide acrylique engagée

## Revendications

1. Procédé de fabrication de chlorure d'acryloyle par réaction de l'acide acrylique sur le phénylchloroforme, en présence d'au moins un acide de Lewis comme catalyseur et d'au moins un agent inhibiteur de polymérisation, caractérisé par le fait que le catalyseur est choisi parmi l'oxyde de zinc et le tétrachlorure de zirconium, et qu'on conduit la réaction à une température d'au moins 105 °C.

2. Procédé selon la revendication 1, caractérisé par le fait qu'on additionne l'acide acrylique au milieu constitué par le phénylchloroforme, le catalyseur et l'inhibiteur de polymérisation, et porté à une température d'au moins 105 °C, le chlorure d'acryloyle formé distillant dès le début de l'introduction de l'acide acrylique.

3. Procédé selon la revendication 2, caractérisé par le fait qu'on additionne l'acide acrylique par le fond du réacteur contenant le milieu constitué par le phénylchloroforme, le catalyseur et l'inhibiteur de polymérisation, le chlorure d'acryloyle formé étant récupéré en sortie d'une colonne de distillation montée sur la sortie du réacteur, à la partie supérieure de celui-ci.

4. Procédé selon l'une des revendications 1 à 3, caractérisé par le fait qu'on conduit la réaction à une température comprise entre 105 °C et 180 °C.

5. Procédé selon l'une des revendications 2 à 4, caractérisé par le fait qu'on maintient la température choisie tout au long de l'addition de l'acide acrylique, puis qu'on l'élève progressivement pour permettre l'achèvement de la distillation du chlorure d'acryloyle.

6. Procédé selon l'une des revendications 1 à 5, caractérisé par le fait qu'on travaille à la pression atmosphérique.

7. Procédé selon l'une des revendications 1 à 6, caractérisé par le fait qu'on conduit la réaction avec un rapport sensiblement molaire de l'acide acrylique au phénylchloroforme.

8. Procédé selon l'une des revendications 1 à 7, caractérisé par le fait qu'on utilise une quantité de catalyseur se situant entre 0,1 et 5% en poids par rapport à la quantité d'acide acrylique mise en jeu.

9. Procédé selon l'une des revendications 1 à 8, caractérisé par le fait qu'on utilise au moins un inhibiteur de polymérisation choisi parmi l'hydroquinone, l'éther méthylique de l'hydroquinone, le ditert.-butyl paracrésol, la phénothiazine et le chlorure de cuivre, à raison de 0,05 à 2% en poids par rapport à la quantité d'acide acrylique mise en jeu.

## Claims

1. Process for the manufacture of acryloyl chloride by reaction of acrylic acid with phenylchloroform, in the presence of at least one Lewis acid as catalyst and of at least one polymerisation inhibiting agent, characterised in that the catalyst is chosen from zinc oxide and zirconium tetrachloride, and in that the reaction is carried out at a temperature of at least 105°C.

2. Process according to Claim 1, characterised in that acrylic acid is added to the mixture consisting of phenylchloroform, the catalyst and polymerisation inhibitor, which is maintained at a temperature of at least 105°C, the acryloyl chloride formed distilling from the beginning of introduction of acrylic acid.

3. Process according to Claim 2, characterised in that acrylic acid is added via the bottom of the reactor containing the mixture consisting of phenylchloroform, the catalyst and the polymerisation inhibitor, the acryloyl chloride formed being recovered at the outlet of a distillation column mounted on the outlet of the reactor, on the upper part of the latter.

4. Process according to one of Claims 1 to 3, characterised in that the reaction is carried out at a temperature of between 105°C and 180°C.

5. Process according to one of Claims 2 to 4, characterised in that the chosen temperature is maintained throughout the addition of acrylic acid and in that it is then progressively raised to enable distillation of the acryloyl chloride to be completed.

6. Process according to one of Claims 1 to 5, characterised in that the reaction is carried out at atmospheric pressure.

7. Process according to one of Claims 1 to 6, characterised in that the reaction is carried out with a substantially molar ratio of acrylic acid to phenylchloroform.

8. Process according to one of Claims 1 to 7, characterised in that an amount of catalyst is used lying between 0.1 and 5 % by weight with respect to the amount of acrylic acid involved.

9. Process according to one of Claims 1 to 8, characterised in that there is used at least one polymerisation inhibitor chosen from hydroquinone, hydroquinone methyl ether, di(tert-butyl)paracresol, phenothiazine and copper chloride, at a concentration of 0.05 to 2 % by weight with respect to the amount of acrylic acid involved.

## Patentansprüche

1. Verfahren zur Herstellung von Acrylsäurechlorid durch Reaktion von Acrylsäure mit Phenylchloroform in Gegenwart mindestens einer Lewis-Säure als Katalysator und mindestens einem Polymerisationsinhibitor, dadurch gekennzeichnet, daß der Katalysator ausgewählt wird aus der Gruppe bestehend aus Zinkoxid und Zirkontetrachlorid, und daß man die Reaktion bei einer Temperatur von mindestens 105°C durchführt.

2. Verfahren nach Anspruch 1, dadurch gekennzeichnet, daß man die Acrylsäure dem von dem Phenylchloroform, dem Katalysator und dem Polymerisationsinhibitor gebildeten und auf eine Temperatur von mindestens 105°C gebrachten Milieu zugibt, wobei das gebildete Acrylsäurechlorid von Beginn des Einbringens der Acrylsäure an destilliert wird.

3. Verfahren nach Anspruch 2, dadurch gekennzeichnet, daß man die Acrylsäure durch den Boden des das von dem Phenylchloroform, dem Katalysator und dem Polymerisationsinhibitor gebildete Milieu enthaltenden Reaktors zugibt, wobei das gebildete Acrylsäurechlorid am Ausgang einer am Auslaß des Reaktors im oberen Teil desselben montierten Destillationskolonne gewonnen wird.

4. Verfahren nach einem der Ansprüche 1 bis 3, dadurch gekennzeichnet, daß man die Reaktion bei einer Temperatur von 105° bis 180°C durchführt.

5. Verfahren nach einem der Ansprüche 2 bis 4, dadurch gekennzeichnet, daß man die gewählte Temperatur während der gesamten Dauer der Zugabe der Acrylsäure aufrechterhält, wonach man die Temperatur nach und nach erhöht, um die Vollendung der Destillation des Acrylsäurechlorids zu ermöglichen.

6. Verfahren nach einem der Ansprüche 1 bis 5, dadurch gekennzeichnet, daß man bei Atmosphärendruck arbeitet.

7. Verfahren nach einem der Ansprüche 1 bis 6, dadurch gekennzeichnet, daß man die Reaktion mit einem im wesentlichen molaren Verhältnis der Acrylsäure zum Phenylchloroform durchführt.

8. Verfahren nach einem der Ansprüche 1 bis 7, dadurch gekennzeichnet, daß man eine Katalysatormenge im Bereich von 0,1 bis 5 Gew.-%, bezogen auf die eingesetzte Acrylsäuremenge, verwendet.

9. Verfahren nach einem der Ansprüche 1 bis 8, dadurch gekennzeichnet, daß man mindestens einen Polymerisationsinhibitor, der ausgewählt ist aus der Gruppe bestehend aus Hydrochinon, Hydrochinonmethylether, ditert.-Butylparakresol, Phenothiazin und Kupferchlorid, in einer Menge von 0,05 bis 2 Gew.-%, bezogen auf die eingesetzte Acrylsäuremenge, verwendet.